# EUROPÄISCHE PATENTSCHRIFT

(11) **EP 1 558 521 B1**
(45) Veröffentlichungstag und Bekanntmachung des Hinweises auf die Patenterteilung: **08.09.2010**
(21) Anmeldenummer: 03778287.7
(22) Anmeldetag: 27.10.2003
(51) Int. Cl.: C01B 7/04

(54) **VERFAHREN ZUR HERSTELLUNG VON CHLOR AUS SALZSÄURE UND EIN DAMIT INTEGRIERTES VERFAHREN ZUR HERSTELLUNG VON ISOCYANATEN**
METHOD FOR PRODUCING CHLORINE FROM HYDROCHLORIC ACID
PROCEDE DE PREPARATION DE CHLORE A PARTIR D'ACIDE CHLORHYDRIQUE

(30) Priorität: 28.10.2002 DE 10250131
(43) Veröffentlichungstag der Anmeldung: 03.08.2005
(73) Patentinhaber: BASF SE, 67056 Ludwigshafen (DE)
(72) Erfinder: WALSDORFF, Christian, 67059 Ludwigshafen (DE); FIENE, Martin, 67150 Niederkirchen (DE); KUHRS, Christian, 69115 Heidelberg (DE); STRÖFER, Eckhard, 68163 Mannheim (DE); HARTH, Klaus, 67317 Altleiningen (DE)
(86) Internationale Anmeldenummer: PCT/EP2003/011910
(87) Internationale Veröffentlichungsnummer: WO 2004/037718

(56) Entgegenhaltungen:
- EP-A- 0 618 170
- EP-A- 0 765 838
- EP-A- 1 099 666
- US-A- 2 312 952
- US-A- 2 542 961
- US-A- 4 394 367
- US-A- 4 774 070

## Beschreibung

Die Erfindung betrifft ein Verfahren zur Herstellung von Chlor aus Salzsäure.

In dem von Deacon 1868 entwickelten Verfahren der katalytischen Chlorwasserstoff-Oxidation wird Chlorwasserstoff mit Sauerstoff in einer exothermen Gleichgewichtsreaktion zu Chlor oxidiert. Durch Überführung von Chlorwasserstoff in Chlor kann die Chlorherstellung von der Natronlaugeherstellung durch Chloralkalielektrolyse entkoppelt werden. Eine solche Entkoppelung ist attraktiv, da weltweit der Chlorbedarf stärker als die. Nachfrage nach Natronlauge wächst. Zudem fällt Chlorwasserstoff in großen Mengen beispielsweise bei Phosgenierungsreaktionen, etwa bei der Isocyanat-Herstellung, als Koppelprodukt an. Der bei der Isocyanatherstellung gebildete Chlorwasserstoff wird überwiegend in der Oxichlorierung von Ethylen zu 1,2-Dichlorethan eingesetzt, das zu Vinylchlorid und schließlich zu PVC weiterverarbeitet wird.

Der in der Deacon-Reaktion eingesetzte Chlorwasserstoff wird üblicher Weise in gasförmiger Form bereitgestellt. Es handelt sich dabei häufig um gasförmigen Chlorwasserstoff, der in anderen Herstellverfahren, beispielsweise bei der Isocyanat-Herstellung, als Koppelprodukt anfällt.

An isolierten Produktionsstandorten kann es jedoch vorkommen, dass kein gasförmiger Chlorwasserstoff aus anderen Prozessen über Gasleitungen zur Verfügung steht. Dann muss auf Salzsäure, beispielsweise aus Kesselwagen, zurückgegriffen werden. Ferner wird ein Verfahren gesucht, mit dem wässrige Salzsäure, die an anderen Standorten anfällt, verwertet werden kann.

US 2,542,961 offenbart ein Verfahren zur Herstellung von Chlor durch katalytische Oxidation von Chlorwasserstoff mit Sauerstoff. Dabei werden wasserfreier Chlorwasserstoff und Sauerstoff in einen Oxidationsreaktor eingeleitet. Aus dem Produktgasgemisch der Chlorwasserstoff-Oxidation wird in einem Chlorwasserstoff-Absorber Chlorwasserstoff mittels azeotroper Salzsäure herausgewaschen, wobei eine Salzsäure höherer Konzentration erhalten wird. Aus dieser wird in einem Chlorwasserstoff-Stripper wasserfreier Chlorwasserstoff wiedergewonnen und in die Chlorwasserstoff-Oxidation zurückgeführt. Daneben wird eine azeotrope Salzsäure zurückgewinnen, welche in den Chlorwasserstoff-Absorber zurückgeführt wird.

EP-A 0 618 170 offenbart ein Verfahren zur Herstellung von Salzsäure aus Chlorwasserstoff, welcher bei der Herstellung von organischen Isocyanaten aus organischen Aminen und Phosgen anfällt

Aufgabe der Erfindung ist es, ein vorteilhaftes Verfahren zur Herstellung von Chlor aus Chlorwasserstoff bereitzustellen, bei dem Chlorwasserstoff nicht gasförmig, sondern zumindest teilweise in Form von Salzsäure eingesetzt wird.

Gelöst wird die Aufgabe durch ein Verfahren zur Herstellung von Chlor aus Salzsäure mit den Schritten:
a) es wird ein Salzsäure-Einsatzstrom I bereitgestellt;
b) es wird ein Salzsäure-Rückstrom II bereitgestellt;
c) in einem Destillationsschritt wird aus dem Salzsäure-Einsatzstrom I und dem Salzsäure-Rückstrom II ein Chlorwasserstoffstrom IV abgetrennt, in einem ersten Teilschritt c1) die Salzsäureströme I und II in einen Chlorwasserstoffstrom IV und einen azeotrop siedenden Salzsäurestrom IIa aufgetrennt, und der azeotrop siedende Salzsäurestrom IIa in einem zweiten Teilschritt c2) in einen Wasserdampfstrom IX und einen Salzsäurestrom IIb höherer Konzentration als derjenigen des Salzsäurestroms 11a aufgetrennt und der Salzsäurestrom IIb in den Teilschritt c1) zurückgeführt, wobei der erste Teilschritt c1) bei einem höheren Druck als der zweite Teilschritt c2) durchgeführt wird;
d) der Chlorwasserstoffstrom IV, ein Sauerstoff enthaltender Strom V und gegebenenfalls ein Sauerstoff enthaltender Rückstrom Va werden in eine Oxidationszone eingespeist und Chlorwasserstoff in Gegenwart eines Katalysators zu Chlor oxidiert, wobei ein Produktgasstrom VI erhalten wird, der Chlor, nicht umgesetzten Sauerstoff, nicht umgesetzten Chlorwasserstoff und Wasserdampf enthält;
e) aus dem Produktgasstrom VI werden in einem Quench- und/oder Absorptionsschritt Chlorwasserstoff und Wasser abgetrennt, wobei ein Gasstrom VII und der Salzsäure-Rückstrom II erhalten wird;
f) gegebenenfalls wird der Gasstrom VII getrocknet;
g) aus dem Gasstrom VII wird ein Sauerstoff enthaltender Strom abgetrennt und gegebenenfalls zumindest teilweise als Sauerstoff enthaltender Rückstrom Va in die Oxidationszone zurückgeführt, wobei ein Chlor enthaltender Produktstrom VIII verbleibt;
h) gegebenenfalls wird der Chlor enthaltende Produktstrom VIII weiter aufgereinigt.

Nach dem erfindungsgemäßen Verfahren ist es ohne weiteres möglich, flüssige Salzsäure-Einsatzströme zu verarbeiten. In dem Destillationsschritt c) fällt am Kopf der Kolonne ein Chlorwasserstoffstrom an, welcher unter dem bei der Kopftemperatur T geltenden Eigendruck steht. Dieser beträgt üblicher Weise ca. 2 bis 20 bar. Der Druck ist ausreichend hoch, um den Chlorwasserstoffstrom in dem Chlorwasserstoff-Oxidationsreaktor einzuspeisen, ohne dass es des Einsatzes zusätzlicher Kompressoren bedarf.

Vorzugsweise wird der Salzsäure-Einsatzstrom I erhalten, indem
a1) ein Chlorwasserstoff enthaltender Einspeisungsgastrom Ia, der nicht wasserlösliche Nebenbestandteile enthalten kann, bereitgestellt wird;
a2) in einem Absorptionsschritt Chlorwasserstoff in Wasser absorbiert wird, wobei der Salzsäure-Einsatzstrom I und gegebenenfalls ein Abgasstrom III enthaltend nicht wasserlöslichen Verunreinigungen erhalten wird.

Der in dem Verfahrenschritt a1) eingesetzte, Chlorwasserstoff enthaltende Einspeisungsgasstrom Ia ist vorzugsweise ein chlorwasserstoffhaltiger Strom, der bei einem Verfahren als Abstrom anfällt, in dem Chlorwasserstoff als Koppelprodukt gebildet wird. Solche Verfahren sind beispielsweise
(1) die Isocyanat-Herstellung aus Phosgen und Aminen,
(2) die Säurechlorid-Herstellung,
(3) die Polycarbonat-Herstellung,
(4) die Herstellung von Vinylchlorid aus Ethylendichlorid,
(5) die Chlorierung von Aromaten.

Der Chlorwasserstoff enthaltende Einspeisungsgasstrom Ia kann Nebenbestandteile enthalten. Üblicherweise enthält er in Wasser nicht lösliche Verunreinigungen, die sowohl organischer als auch anorganischer Natur sein können. Organische Verunreinigungen sind beispielsweise Kohlenwasserstoffe oder Chlorkohlenwasserstoffe.

Typische Kohlenwasserstoffe, die in den erfindungsgemäß eingesetzten Chlorwasserstoff enthaltenden Einsatzgasströmen enthalten sein können, umfassen Aromaten wie Benzol, Toluol, Xylole und C₆-C₁₂-Aliphaten. Typische Chlorkohlenwasserstoffe umfassen Phosgen, Tetrachlorkohlenstoff, Vinylchlorid und Dichlorethan. Die Kohlenwasserstoffe und Chlorkohlenwasserstoffe können in Mengen bis 20 Vol.-%, im allgemeinen bis 30 000 ppm, bevorzugt in Mengen bis 10 000 ppm und insbesondere in Mengen von 100 bis 3000 ppm enthalten sein. Als anorganische Nebenbestandteile können beispielsweise Kohlenmonoxid, Kohlendioxid, Stickstoff sowie weitere Inertgase, im allgemeinen in Mengen bis 10 Vol.-%, vorzugsweise in Mengen bis zu 1 Vol.-%, enthalten sein.

In einem Absorptionsschritt a2) wird Chlorwasserstoff in Wasser absorbiert, wobei ein Strom aus verdünnter Salzsäure als Salzsäure-Einspeisungsstrom I und gegebenenfalls ein Abgasstrom III enthaltend die nicht wasserlöslichen Verunreinigungen erhalten wird. Der Absorptionsschritt a2) wird üblicherweise mittels eines Gaswäschers durchgeführt, wobei Chlorwasserstoff aus dem Einsatzgasstrom als wässrige Salzsäure herausgewaschen wird.

In einem Destillationsschritt c) wird aus dem Salzsäure-Einsatzstrom I und dem Salzsäure-Rückstrom II ein Chlorwasserstoffstrom IV abgetrennt. Dies kann dadurch geschehen, dass die Salzsäureströme I und II vereinigt und gemeinsam in einer Destillationskolonne in einen Chlorwasserstoff IV und einen Strom IIa aus azeotrop siedender Salzsäure aufgetrennt werden. Üblicherweise wird die Destillation bei einem Druck von 1 bis 20 bar, bevorzugt bei einem Druck von 2 bis 15 bar durchgeführt. Der Destillationsschritt c) kann in üblichen Destillationskolonnen durchgeführt werden. Diese sollten korrosionsbeständig sein. Am Kopf der Destillationskolonne wird ein im Wesentlichen wasserfreier Chlorwasserstoffstrom IV erhalten. Am Sumpf der Kolonne wird ein Strom IIa aus azeotrop siedender verdünnter Salzsäure erhalten, wobei die HCl-Konzentration der azeotrop siedenden Salzsäure abhängig vom Kolonnendruck ist. Üblicherweise beträgt die HCl-Konzentration der azeotrop siedenden Salzsäure des Stroms IIa 10 bis 25 Gew.-%.

Der Chlorwasserstoffstrom IV wird in die katalytische Chlorwasserstoff-Oxidation gerührt. Der Druck, unter dem der Chlorwasserstoffstrom IV bei Verlassen der Destillationskolonne steht, beträgt üblicher Weise 2 bis 30 bar und ist damit in der Regel ausreichend hoch, so dass bei der Rückführung des Chlorwasserstoffs in die katalytische Chlorwasserstoff-Oxidation auf einen Kompressor verzichtet werden kann.

In einem Oxidationsschritt d) werden der Chlorwasserstoffstrom IV, ein Sauerstoff enthaltender Strom V und gegebenenfalls ein Sauerstoff enthaltender Rückstrom Va in eine Oxidationszone eingespeist und wird Chlorwasserstoff in Gegenwart eines Katalysators zu Chlor oxidiert, wobei ein Produktgasstrom VI erhalten wird, der Chlor, nicht umgesetzten Sauerstoff, nicht umgesetzten Chlorwasserstoff und Wasserdampf enthält.

In dem auch als Deacon-Prozess bekannten katalytischen Verfahren wird Chlorwasserstoff mit Sauerstoff in einer exothermen Gleichgewichtsreaktion zu Chlor oxidiert, wobei Wasserdampf anfällt. Übliche Reaktionstemperaturen liegen zwischen 150 und 500°C, übliche Reaktionsdrucke liegen zwischen 1 und 25 bar. Ferner ist es zweckmäßig, Sauerstoff in überstöchiometrischen Mengen einzusetzen. Üblich ist beispielsweise ein zwei- bis vierfacher Sauerstoff-Überschuss. Da keine Selektivitätsverluste zu befürchten sind, kann es wirtschaftlich vorteilhaft sein, bei relativ hohen Drücken und dementsprechend bei gegenüber Normaldruck längeren Verweilzeiten zu arbeiten.

Geeignete Katalysatoren enthalten beispielsweise Rutheniumoxid, Rutheniumchlorid oder andere Rutheniumverbindungen auf Siliziumdioxid, Aluminiumoxid, Titandioxid oder Zirkondioxid als Träger. Geeignete Katalysatoren können beispielsweise durch Aufbringen von Rutheniumchlorid auf den Träger und anschließendes Trocknen oder Trocknen und Calcinieren erhalten werden. Geeignete Katalysatoren können ergänzend zu oder an Stelle einer Rutheniumverbindung auch Verbindungen anderer Edelmetalle, beispielsweise Gold, Palladium, Platin, Osmium, Iridium, Silber, Kupfer oder Rhenium enthalten. Geeignete Katalysatoren können ferner Chrom(III)oxid enthalten.

Geeignet sind ferner Katalysatoren, welche auf einem Träger 0,001 bis 30 Gew.-% Gold, 0 bis 3 Gew.-% eines oder mehrerer Erdalkalimetalle, 0 bis 3 Gew.-% eines oder mehrerer Alkalimetalle, 0 bis 10 Gew.-% eines oder mehrerer Seltenerdmetalle und 0 bis 10 Gew.-% eines oder mehrerer weiterer Metalle, ausgewählt aus der Gruppe bestehend aus Ruthenium, Palladium, Platin, Osmium, Iridium, Silber, Kupfer und Rhenium, jeweils bezogen auf das Gesamtgewicht des Katalysators, enthalten.

Derartige Gold enthaltende Trägerkatalysatoren weisen, insbesondere bei Temperaturen von ≤ 250 °C, bei der Chlorwasserstoff-Oxidation eine höhere Aktivität als die Ruthenium enthaltenden Katalysatoren des Standes der Technik auf.

Übliche Reaktionsapparate, in denen die katalytische Chlorwasserstoff-Oxidation durchgeführt wird, sind ein Festbett- oder Wirbelbettreaktor. Die Chlorwasserstoff-Oxidation kann mehrstufig durchgeführt werden.

Die katalytische Chlorwasserstoff-Oxidation kann adiabat oder bevorzugt isotherm oder annähernd isotherm, diskontinuierlich, bevorzugt kontinuierlich als Fließ- oder Festbettverfahren, bevorzugt als Festbettverfahren, besonders bevorzugt in Rohrbündelreaktoren an Heterogenkatalysatoren bei Realctortemperaturen von 180 bis 500°C, bevorzugt 200 bis 400°C, besonders bevorzugt 220 bis 350°C und einem Druck von 1 bis 25 bar, bevorzugt 1,2 bis 20 bar, besonders bevorzugt 1,5 bis 17 bar und insbesondere 2,0 bis 15 bar durchgeführt werden.

Bei der isothermen oder annähernd isothermen Fahrweise können auch mehrere, also 2 bis 10, bevorzugt 2 bis 6, besonders bevorzugt 2 bis 5, insbesondere 2 bis 3 in Reihe geschaltete Reaktoren mit zusätzlicher Zwischenkühlung eingesetzt werden. Der Sauerstoff kann entweder vollständig zusammen mit dem Chlorwasserstoff vor dem ersten Reaktor oder über die verschiedenen Reaktoren verteilt zugegeben werden. Diese Reihenschaltung einzelner Reaktoren kann auch in einem Apparat zusammengeführt werden.

Eine bevorzugte Ausführungsform besteht darin, dass man eine strukturierte Katalysatorschüttung einsetzt, bei der die Katalysatoraktivität in Strömungsrichtung ansteigt. Eine solche Strukturierung der Katalysatorschüttung kann durch unterschiedliche Tränkung der Katalysatorträger mit Aktivmasse oder durch unterschiedliche Verdünnung des Katalysators mit einem Inertmaterial erfolgen. Als Inertmaterial können beispielsweise Ringe, Zylinder oder Kugeln aus Titandioxid, Zirkondioxid oder deren Gemischen, Aluminiumoxid, Steatit, Keramik, Glas, Graphit oder Edelstahl eingesetzt werden. Beim bevorzugten Einsatz von Katalysatorformkörpern sollte das Inertmaterial bevorzugt ähnliche äußeren Abmessungen haben.

Als Katalysatorformköiper eignen sich beliebige Formen, bevorzugt sind Tabletten, Ringe, Zylinder, Sterne, Wagenräder oder Kugeln, besonders bevorzugt sind Ringe, Zylinder oder Stemstränge.

Als Heterogenkatalysatoren eignen sich insbesondere Rutheniumverbindungen oder Kupferverbindungen auf Trägermaterialen, die auch dotiert sein können, bevorzugt sind gegebenenfalls dotierte Rutheniumkatalysatoren. Als Trägermaterialen eignen sich beispielsweise Siliciumdioxid, Graphit, Titandioxid mit Rutil- oder Anatas-Struk-tur, Zirkondioxid, Aluminiumoxid oder deren Gemische, bevorzugt Titandioxid, Zirkondioxid, Aluminiumoxid oder deren Gemische, besonders bevorzugt γ- oder δ-Aluminiumoxid oder deren Gemische.

Die Kupfer- bzw. die Rutheniumträgerkatalysatoren können beispielsweise durch Tränkung des Trägermaterials mit wässrigen Lösungen von CuCl₂ bzw. RuCl₃ und gegebenenfalls eines Promotors zur Dotierung, bevorzugt in Form ihrer Chloride, erhalten werden. Die Formgebung des Katalysators kann nach oder bevorzugt vor der Tränkung des Trägermaterials erfolgen.

Zur Dotierung eignen sich als Promotoren Alkalimetalle wie Lithium, Natrium, Kalium, Rubidium und Cäsium, bevorzugt Lithium, Natrium und Kalium, besonders bevorzugt Kalium, Erdalkalimetalle wie Magnesium, Calcium, Strontium und Barium, bevorzugt Magnesium und Calcium, besonders bevorzugt Magnesium, Seltenerdmetalle wie Scandium, Yttrium, Lanthan, Cer, Praseodym und Neodym, bevorzugt Scandium, Yttrium, Lanthan und Cer, besonders bevorzugt Lanthan und Cer, oder deren Gemische.

Die Formkörper können anschließend bei Temperaturen von 100 bis 400°C, bevorzugt 100 bis 300°C beispielsweise unter einer Stickstoff-, Argon- oder Luftatmosphäre getrocknet und gegebenenfalls calciniert werden. Bevorzugt werden die Formkörper zunächst bei 100 bis 150°C getrocknet und anschließend bei 200 bis 400°C calciniert.

Der Umsatz an Chlorwasserstoff im einfachen Durchgang kann auf 15 bis 90 %, bevorzugt 40 bis 85 %, besonders bevorzugt 50 bis 80 % begrenzt werden. Nicht umgesetzter Chlorwasserstoff kann nach Abtrennung teilweise oder vollständig in die katalytische Chlorwasserstoff-Oxidation zurückgeführt werden. Das Volumenverhältnis von Chlorwasserstoff zu Sauerstoff am Reaktoreintritt liegt in der Regel zwischen 1:1 1 und 20:1, bevorzugt zwischen 2:1 und 8:1, besonders bevorzugt zwischen 2:1 und 5:1.

In einem Quench- und/oder Absorptionsschritt e) werden aus dem Produktgasstrom VI Chlorwasserstoff und Wasser abgetrennt, wobei ein Gasstrom VII und ein Strom II aus verdünnter Salzsäure erhalten werden. Als Absorptionsmittel geeignet sind Wasser und jede verdünnte Salzsäure, die nicht an Chlorwasserstoff gesättigt ist. Vorzugsweise wird Wasser als Absorptionsmittel eingesetzt. Die Absorptionstemperatur beträgt üblicherweise von 0 bis 150 °C, vorzugsweise von 30 bis 100°C, der Absorptionsdruck beträgt üblicherweise von 0,5 bis 20 bar, vorzugsweise von 1 bis 10 bar.

Es wird ein Gasstrom VII erhalten, der Chlor und Sauerstoff enthält oder im wesentlichen aus diesen Gasen besteht. Dieser enthält üblicherweise noch Spuren von Feuchtigkeit. Üblicherweise wird daher eine Trocknungsstufe f) durchgeführt, in der der Gasstrom aus Chlor und Sauerstoff durch Inkontaktbringen mit geeigneten Trocknungsmitteln von Feuchtigkeitsspuren befreit wird. Geeignete Trocknungsmittel sind beispielsweise konzentrierte Schwefelsäure, Molsiebe oder hygroskopische Adsorbentien.

In einem Abtrennschritt g) wird aus dem Gasstrom VII ein Sauerstoff enthaltender Strom abgetrennt, der gegebenenfalls zumindest teilweise als Sauerstoff enthaltender Rückstrom Va in die Oxidationszone zurückgeführt wird. Es verbleibt ein Chlor enthaltender Produktstrom VIII.

Die Sauerstoff-Abtrennung erfolgt vorzugsweise durch Destillation, üblicherweise bei einer Temperatur im Bereich von -20 bis +50 °C und einem Druck im Bereich von 1 bis 20 bar in einer Destillationskolonne mit 10 bis 100 theoretischen Böden.

Der Chlor enthaltende Produktstrom VIII kann weiter aufgereinigt werden.

In einer bevorzugten Ausführungform wird der chlorwasserstoffhaltige Einsatzgasstrom I bei der Isocyanatsynthese aus Phosgen und primären Aminen gewonnen.

In einer besonders bevorzugten Ausführungsform der Erfindung wird der chlorwasserstoffhaltige Einsatzgasstrom I bei der Isocyanatsynthese aus Phosgen und primären Aminen gewonnen, und wird der Chlor enthaltende Produktgasstrom VIII zur Herstellung des Phosgens eingesetzt, das nachfolgend mit den primären Aminen zu Isocyanaten umgesetzt wird.

Gegenstand der vorliegenden Erfindung ist daher auch ein integriertes Verfahren zur Herstellung von organischen Isocyanaten mit den Schritten
i) ein Kohlenmonoxid enthaltender Einspeisungsgassstrom X, ein Chlor enthaltender Rückstrom VIII und gegebenenfalls ein Chlor enthaltender Ergänzungsstrom VIII werden bereitgestellt;
ii) in einem Phosgen-Syntheseschritt werden die Ströme X, VIII und gegebenenfalls VIIIa zu einem Phosgen enthaltenden Gasstrom XI umgesetzt;
iii) in einem Isocyant-Syntheseschritt wird der Phosgen enthaltende Gastrom XI mit einem oder mehreren primären Aminen zu dem oder den entsprechenden Isocyanaten und Chlorwasserstoff umgesetzt, wobei ein Chlorwasserstoff und nicht umgesetztes Phosgen enthaltender Gasstrom XII und ein Isocyanat enthaltender Produktstrom XIII gewonnen werden;
iv) aus dem Chlorwasserstoff und nicht umgesetztes Phosgen enthaltenden Gasstrom XII wird in einem Auftrennschritt ein Chlorwasserstoff und gegebenenfalls nicht wasserlösliche Verunreinigungen enthaltender Gasstrom Ia und ein Phosgen enthaltender Strom XIV gewonnen, wobei der Phosgen enthaltende Strom XIV gegebenenfalls in den Isocyanat-Syntheseschritt iii) zurückgeführt wird;
v) in einem Absorptionsschritt wird Chlorwasserstoff aus dem Gasstrom Ia in Wasser absorbiert, wobei ein Salzsäure-Einsatzstrom I aus verdünnter Salzsäure und gegebenenfalls ein Abgasstrom III enthaltend die nicht wasserlöslichen Verunreinigungen erhalten wird;
vi) aus dem Salzsäure-Einsatzstrom I und einem Salzsäure-Rückstrom II wird in einem Destillationsschritt ein Chlorwasserstoffstrom IV abgetrennt, entsprechend dem oben beschriebenen Destillationsschritt c);
vii) der Chlorwasserstoffstrom IV, ein Sauerstoff enthaltender Strom V und gegebenenfalls ein Sauerstoff enthaltender Rückstrom Va werden in eine Oxidationszone eingespeist und Chlorwasserstoff in Gegenwart eines Katalysators zu Chlor oxidiert, wobei ein Produktgasstrom VI erhalten wird, der Chlor, nicht umgesetzten Sauerstoff, nicht umgesetzten Chlorwasserstoff und Wasserdampf enthält;
viii)aus dem Produktgasstrom VI werden in einem Absorptionsschritt Chlorwasserstoff und Wasser abgetrennt, wobei ein Gasstrom VII und ein Salzsäure-Rückstrom II aus verdünnter Salzsäure erhalten wird;
ix) der Gasstrom VII wird getrocknet;
x) aus dem Gasstrom VII wird ein Sauerstoff enthaltender Strom abgetrennt und gegebenenfalls zumindest teilweise als Sauerstoff enthaltender Rückstrom Va in die Oxidationszone zurückgeführt, wobei ein Chlor enthaltender Produktstrom VIII verbleibt;
xi) der Chlor enthaltende Produktstrom VIII wird, gegebenenfalls nach Aufreinigung, als Chlor enthaltender Rückstrom VIII in Schritt i) eingesetzt.

In einem Schritt i) wird ein Kohlenmonoxid enthaltender Einspeisungsgassstrom X, ein Chlor enthaltende Strom VIII als Rückstrom VII und gegebenenfalls ein Chlor enthaltender Ergänzungsstrom VIIIa, der Chlorverluste ausgleichen soll, bereitgestellt.

In einem Phosgensyntheseschritt ii) werden die Ströme X, VIII und gegebenenfalls VIIIa zu einem Phosgen enthaltenden Gasstrom XI umgesetzt. Verfahren zur Herstellung von Phosgen sind in Ullmanns Enzyklopädie der Industriellen Chemie, 3. Aufl., Bd. 13, Seite 494 - 500 beschrieben. So kann Phosgen durch Überleiten von Kohlenmonoxid und Chlor über Aktivkohle erhalten werden.

In einem Isocyanat-Syntheseschritt iii) wird der Phosgen enthaltende Gasstrom XI mit einem oder mehreren primären Aminen zu dem oder den entsprechenden Isocyanaten und Chlorwasserstoff umgesetzt, wobei ein Chlorwasserstoff und nicht umgesetztes Phosgen enthaltender Gasstrom XII und ein Isocyanat enthaltender Produktstrom XIII gewonnen werden. Diese Reaktion wird auch als Phosgenierung der Amine bezeichnet. Die eingesetzten Amine weisen mindestens eine, bevorzugt zwei, gegebenenfalls auch drei oder mehr primäre Aminogruppen auf.

Die im Rahmen des erfindungsgemäßen Verfahrens stattfindende Isocyanat-Herstellung wird in einer dem Fachmann bekannten Weise durch Umsetzung eines Amins oder eines Gemischs aus zwei oder mehr Aminen mit Phosgen in überstöchiometrischer Menge durchgeführt. Anwendbar sind grundsätzlich alle Verfahren, bei denen ein primäres Amin oder ein Gemisch aus zwei oder mehr primären Aminen mit einer oder mehreren primären Aminogruppen mit Phosgen unter Bildung einer oder mehrerer Isocyanate mit einer oder mehreren Isocyanatgruppen umgesetzt wird.

In einer bevorzugten Ausführungsform der Erfindung wird die Phosgenierung des oder der Amine in einem Lösemittel oder Lösungsmittelgemisch durchgeführt. Als Lösemittel können alle für die Herstellung von Isocyanaten geeigneten Lösemittel eingesetzt werden. Vorzugsweise sind dies inerte aromatische, aliphatische oder alicyclische Kohlenwasserstoffe oder deren halogenierte Derivate. Beispiele für solche Lösemittel sind aromatische Verbindungen wie Mono- oder Dichlorbenzol, beispielsweise o-Dichlorbenzol, Toluol, Xylole, Naphthalinderivate wie Tetralin oder Decalin, Alkane mit etwa 5 bis etwa 12 C-Atomen wie Hexan, Heptan, Octan, Nonan oder Decan, Cycloalkane wie Cyclohexan, weitgehend inerte Ester und Ether wie Ethyl- oder Butylacetat, Tetrahydrofuran, Dioxan oder Diphenylether. Es kann auch ein Teilstrom des produzierten Isocyanats als Lösungsmittel- oder Lösungsmittelbestandteil zurückgeführt werden.

Als Amine eignen sich prinzipiell alle primären Amine, die in geeigneter Weise mit Phosgen zu Isocyanaten reagieren können. Geeignet sind prinzipiell alle linearen oder verzweigten, gesättigten oder ungesättigten aliphatischen oder cycloaliphatischen oder aromatischen primären Mono- oder Polyamine, die mit Phosgen zu Isocyanaten umgesetzt werden können. Beispiele für geeignete Amine sind 1,3-Propylendiamin, 1,4-Butylendiamin, 1,5-Pentamethylendiamin, 1,6-Hexamethylendiamin und die entsprechenden höheren Homologen dieser Reihe, Isophorondiamin (IPDA), Cyclohexylendiamin, Cyclohexylamin, Anilin, Phenylendiamin, p-Toluidin, 1,5-Naphtylendiamin, 2,4- oder 2,6-Toluylendiamin oder deren Gemische, 4,4'-, 2,4'- oder 2,2'-Diphenylmethandiamin oder deren Gemische, sowie höhermolekulare isomere, oligomere oder polymere Derivate der obengenannten Amine und Polyamine.

In einer bevorzugten Ausführungsform der vorliegenden Erfindung werden als Amine die isomeren primären Diphenylmethandiamine (MDA) bzw. deren oligomere oder polymere Derivate eingesetzt, also die Amine der Diphenylmethandiamin-Reihe. Diphenylmethandiamin, dessen Oligomere oder Polymere werden beispielsweise durch Kondensation von Anilin mit Formaldehyd erhalten. Auch solche Oligo- oder Polyamine oder deren Gemische werden im Rahmen einer bevorzugten Ausführungsform der Erfindung eingesetzt. Bevorzugte Amine sind weiterhin Hexamethylendiamin, Toluylendiamin und Isophorondiamin.

Die Umsetzung des Phosgens mit den obengenannten Aminen kann kontinuierlich oder diskontinuierlich in einer oder mehreren Stufen erfolgen. Wird eine einstufige Umsetzung durchgeführt, so erfolgt diese Umsetzung vorzugsweise bei etwa 40 bis 200 °C, beispielsweise bei etwa 90 bis 180 °C.

In einer bevorzugten Ausführungsform der Erfindung wird die Umsetzung zweistufig durchgeführt. Hierbei wird in einer ersten Stufe, die auch als Kaltphosgenierung bezeichnet wird, die Umsetzung des Phosgens mit dem oder den Aminen bei einer Temperatur von 0 bis 160 °C, beispielsweise von 20 bis 130 °C durchgeführt, wobei für die Reaktion zwischen Amin und Phosgen eine Zeitspanne von etwa 0,5 min bis 2 h eingeräumt wird. Anschließend wird in einer zweiten Stufe, die auch als Heißphosgenierung bezeichnet wird, die Temperatur innerhalb einer Zeitspanne von im allgemeinen etwa 1 min bis 5 h, beispielsweise innerhalb von etwa 1 min bis 3 h, auf 60 bis 190 °C, insbesondere 70 bis 170 °C, erhöht.

Während der Umsetzung kann in einer weiteren Ausführungsform der Erfindung erhöhter Druck angelegt werden, im allgemeinen bis zu 100 bar oder weniger, vorzugsweise 1 bar bis etwa 50 bar, besonders bevorzugt 2 bar bis 25 bar, insbesondere. 3 bar bis 12 bar. In einer weiteren Ausführungsform der Erfindung wird bei etwa 1 bar (Umgebungsdruck) gearbeitet. In einer weiteren Ausführungsform wird bei gegenüber dem Umgebungsdruck reduziertem Druck gearbeitet.

Es wird ein Isocyanate enthaltender Produktstrom XIII gewonnen, aus dem nachfolgend die gebildeten Isocyanate abgetrennt und gegebenenfalls gereinigt werden.

Überschüssiges Phosgen kann im Anschluss an die Umsetzung bei einer Temperatur von 50 bis 180°C entfernt werden. Die Entfernung des Lösemittels erfolgt vorzugsweise unter vermindertem Druck, beispielsweise bei einem Druck von 500 mbar oder weniger, bevorzugt von 100 mbar oder weniger. Im Allgemeinen werden dabei die verschiedenen Lösemittel-Komponenten in der Reihe der Siedepunkte abgetrennt, wobei auch die Abtrennung von Gemischen der verschiedenen Komponenten in einer einzigen Verfahrensstufe möglich ist. Anschließend kann das erhaltene Isocyanat fraktioniert werden.

In einem Auftrennschritt iv) wird aus dem Chlorwasserstoff und nicht umgesetztes Phosgen enthaltenden Gasstrom XII ein Chlorwasserstoff und gegebenenfalls ein nicht wasserlösliche Verunreinigungen enthaltender Gasstrom Ia und ein Phosgen enthaltender Strom XIV gewonnen, wobei der Phosgen enthaltende Strom XIV gegebenenfalls in den Isocyanat-Syntheseschritt iii) zurückgeführt wird. Chlorwasserstoff fällt bei der Umsetzung iii) von Phosgen mit Amin üblicherweise gasförmig im Gemisch mit Phosgen und typischer Weise geringen Mengen weiterer Gase wie Kohlenmonoxid, Kohlendioxid, Stickstoff und Spuren von in der Isocyanat-Herstellung eingesetzten Lösemitteln an. Phosgen und schwersiedende Nebenbestandteile können durch Destillation abgetrennt werden. Es wird ein im wesentlichen Chlorwasserstoff enthaltender Strom erhalten. Darin enthaltene Spuren organischer Verbindungen wie Phosgen und Lösemittelreste können in einer nachgeschalteten Reinigungsstufe durch Absorption, Adsorption, Destillation oder Extraktion entfernt werden.

Einfacher ist es jedoch, zur Reinigung den Chlorwasserstoffstrom in Wasser oder verdünnter Salzsäure zu absorbieren, wobei die nicht wasserlöslichen Gasbestandteile in dem Restgasstrom verbleiben.

Dementsprechend wird in einem Absorptionsschritt v) Chlorwasserstoff aus dem Gasstrom Ia in Wasser absorbiert, wobei ein Strom I aus verdünnter Salzsäure und gegebenenfalls ein Abgasstrom III enthaltend die nicht wasserlöslichen Verunreinigungen erhalten wird.

Vor Durchführung des Absorptionsschrittes v) kann der Chlorwasserstoff enthaltende Strom Ia durch Überleiten über ein Reinigungsbett und Absorption von darin enthaltenen Lösemittelresten an dem Reinigungsbett vorgereinigt werden. Das Reinigungsbett besteht dabei aus geeigneten Absorbentien, vorzugsweise in stückiger Form wie Kugeln, Extrudate oder Tabletten. Geeignete Materialien, die als Absorbentien in Frage kommen, sind beispielsweise Aktivkohle, Aluminiumoxid, Titanoxid, Siliziumdioxid, Eisenoxid, Zeolithe und Molsiebe. Geeignete Materialien können auch Metalloxide oder Metallhalogenide, wie Kupfer- oder Rutheniumoxide oder -halogenide bzw. deren Gemische, auf einem Träger aus einem feuerfesten anorganischen Material wie Aluminiumoxid, Titanoxid oder Siliziumdioxid enthalten. Bevorzugte Absorbentien sind Aluminiumoxid, Aktivkohle und Tonerden.

Es wird ein Strom I aus verdünnter Salzsäure erhalten. Auch dieser Strom kann nochmals gereinigt werden. Die nachfolgenden Schritte vi) bis xi) des erfindungsgemäßen Verfahrens zur Herstellung von organischen Isocyanaten entsprechen den oben beschriebenen Schritten c) bis g) des erfindungsgemäßen Verfahrens zur Herstellung von Chlor.

Die Erfindung wird durch die Figuren näher erläutert.

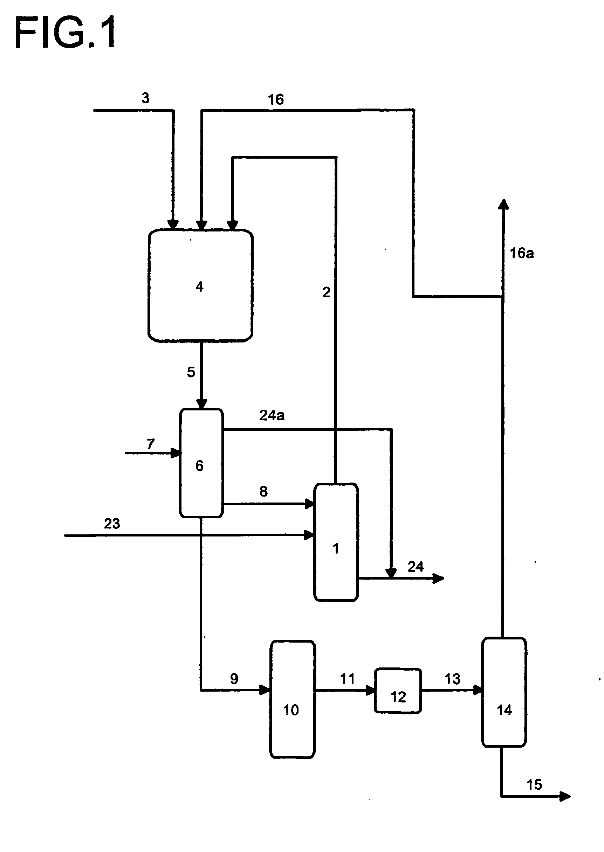

Figur 1 zeigt beispielhaft eine Variante des verfahrens eiher Chlor-Herstellung.

Ein Sauerstoff enthaltender Einspeisungsgasstrom 3, ein Sauerstoff enthaltender Rückstrom 16 und ein Chlorwasserstoff enthaltender Strom 2 werden in den Chlorwasserstoff-Oxidationsreaktor 4 eingespeist, in dem Chlorwasserstoff katalytisch zu Chlor oxidiert wird. Als Sauerstoff enthaltender Strom können beispielsweise reiner Sauerstoff, 94 vol.-%iger Sauerstoff aus einer Druckwechselabsorption (technisch reiner Sauerstoff) oder mit Sauerstoff angereicherte Luft eingesetzt werden. Es wird ein Produk-tgasstrom 5 erhalten, der Chlor, nicht umgesetzten Sauerstoff, nicht umgesetzten Chlorwasserstoff und Wasserdampf enthält. Der Produktgasstrom 5 wird in einen Phasenkontaktapparat 6 eingeleitet und dort mit Wasser 7 in Kontakt gebracht, wobei ein Strom 8 aus verdünnter Salzsäure erhalten wird. Ein extern zugeführter Strom 23 aus verdünnter Salzsäure und der Strom 8 aus verdünnter Salzsäure werden in eine erste Destillationskolonne 1 eingespeist und bei einem Druck p1 destilliert, bei dem als Kopfprodukt Chlorwasserstoff 2 und als Sumpfprodukt bei dem Druck p1 azeotrop siedende Salzsäure 24 erhalten wird. Der Chlorwasserstoffstrom 2 wird als Rückstrom in den Oxidationsreaktor 4 zurückgeführt. Ein Teil der Salzsäure 24 kann als zusätzlicher Absorptionsmittelstrom 24a in den Phasenkontaktapparat 6 eingespeist werden. Den Phasenkontak-tapparat 6 verlässt ein von Chlorwasserstoff befreiter Gasstrom 9 aus Chlor, Sauerstoff und Wasserdampf, der einer Trocknungsstufe 10 zugeleitet wird. In der Trocknungsstufe 10 wird der Gasstrom 9 mit einem geeigneten Absorptionsmittel wie Schwefelsäure, Molsieben oder weiteren hygroskopischen Adsorbentien in Kontakt gebracht und so von Wasserspuren befreit. Optional ist der Trocknungsstufe 10 ein sogenannter De-Mister 12 nachgeschaltet, in dem der getrocknete Gasstrom 10 von mitgerissenen Flüssigkeitspartikeln befreit wird. Ein De-Mister ist bevorzugt vorgesehen, falls die Trocknungsstufe 10 eine Absorption an Schwefelsäure beinhaltet. Der getrocknete und gegebenenfalls von Flüssigkeitspartikeln befreite Gasstrom 13 aus Chlor und Sauerstoff wird der Destillationsstufe 14 zugeführt, in der Sauerstoff abgetrennt und als Rückstrom 16 in den Chlorwasserstoff-Oxidationsreaktor zurückgeführt wird. Es wird ein Produktstrom 15 aus Chlor erhalten. Um die Aufpegelung von inerten Gasbestandteilen wie Stickstoff, Argon (gegebenenfalls aus dem Sauerstoff enthaltenden Einspeisungsstrom 3, falls kein reiner Sauerstoff eingesetzt wird) oder Kohlendioxid (gegebenenfalls aus der Verbrennung der Kohlenwasserstoffe oder Chlorkohlenwasserstoffe) zu vermeiden, ist ein Purge-Strom 16a vorgesehen.

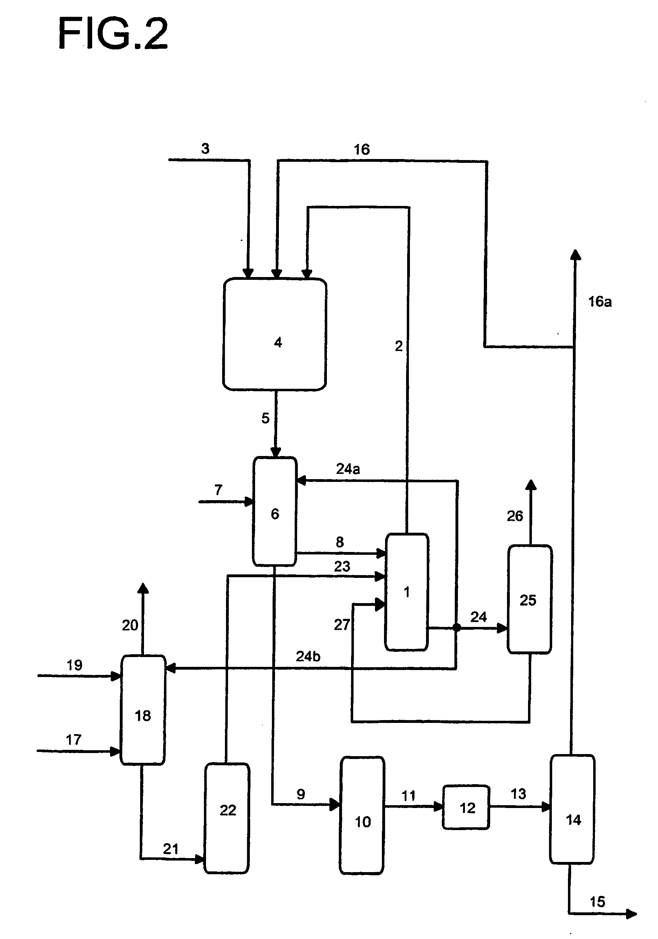

Figur 2 zeigt beispielhaft eine Variante des erfmdungsgemäßen Verfahrens der Chlor-Herstellung.

Ein Sauerstoff enthaltender Einspeisungsgasstrom 3, ein Sauerstoff enthaltender Rückstrom 16 und ein Chlorwasserstoff enthaltender Rückstrom 2 werden in den Chlorwasserstoff-Oxidationsreaktor 4 eingespeist, in dem Chlorwasserstoff katalytisch zu Chlor oxidiert wird. Als Sauerstoff enthaltender Strom können beispielsweise reiner Sauerstoff, 94 vol.-%iger Sauerstoff aus einer Druckwechselabsorption (technisch reiner Sauerstoff) oder mit Sauerstoff angereicherte Luft eingesetzt werden. Es wird ein Produktgasstrom 5 erhalten, der Chlor, nicht umgesetzten Sauerstoff, nicht umgesetzten Chlorwasserstoff und Wasserdampf enthält. Der Produktgasstrom 5 wird in einen Phasenkontaktapparat 6 eingeleitet und dort mit Wasser 7 in Kontakt gebracht, wobei ein Strom 8 aus verdünnter Salzsäure erhalten wird. Ein Chlorwasserstoff und gasförmige, nicht wasserlösliche Nebenbestandteile enthaltender Strom 17 wird in einen Phasenkontaktapparat 18, vorzugsweise einen Gaswäscher, eingeleitet und mit Wasser 19 in Kontakt gebracht, wobei ein Strom 21 aus verdünnter Salzsäure und ein die gasförmigen Nebenbestandteile enthaltender Abgasstrom 20 erhalten wird. Der Salzsäurestrom 21 kann in der Strippkolonne 22 von nicht wasserlöslichen, in der Salzsäure dispergierten Spuren-Verunreinigungen mit Wasserdampf frei gestrippt werden, wobei ein gereinigter Salzsäurestrom 23 erhalten wird. Die Ströme 8 und 23 aus verdünnter Salzsäure werden in eine erste Destillationskolonne 1 eingespeist und bei einem Druck p1 destilliert, bei dem als Kopfprodukt Chlorwasserstoff 2 und als Sumpfprodukt bei dem Druck p1 azeotrop siedende Salzsäure 24 erhalten wird. Der Chlorwasserstoffstrom 2 wird als Rückstrom in den Oxidationsreaktor 4 zurückgeführt. Der azeotrop siedende Chlorwasserstoffstrom 24 wird einer zweiten Destillationskolonne 25 zugeführt und bei einem Druck p2 < p1 destilliert, wobei am Kopf der Kolonne Wasserdampf 26 erhalten wird und am Kolonnensumpf eine bei dem Druck p2 azeotrop siedende Salzsäure 27, deren Konzentration höher ist als die Konzentration der Salzsäure 24. Die Salzsäure 27 wird in die erste Destillationskolonne 1 zurückgeführt. Ein Teil der Salzsäure 24 kann als zusätzlicher Absorptionsmittelstrom 24a in den Phasenkontaktapparat 6 eingespeist werden, ein anderer Teil als zusätzlicher Absorptionsmittelstrom 24b in den Phasenkontaktapparat 18 eingespeist werden. Den Phasenkontaktapparat 6 verlässt ein von Chlorwasserstoff befreiter Gasstrom 9 aus Chlor, Sauerstoff und Wasserdampf, der einer Trocknungsstufe 10 zugeleitet wird. In der Trocknungsstufe 10 wird der Gasstrom 9 mit einem geeigneten Absorptionsmittel wie Schwefelsäure, Molsieben oder weiteren hygroskopischen Adsorbentien in Kontakt gebracht und so von Wasserspuren befreit. Optional ist der Trocknungsstufe 10 ein sogenannter De-Mister 12 nachgeschaltet, in dem der getrocknete Gasstrom 10 von mitgerissenen Flüssigkeitspartikeln befreit wird. Ein De-Mister ist bevorzugt vorgesehen, falls die Trocknungsstufe 10 eine Absorption an Schwefelsäure beinhaltet. Der getrocknete und gegebenenfalls von Flüssigkeitspartikeln befreite Gasstrom 13 aus Chlor und Sauerstoff wird der Destillationsstufe 14 zugeführt, in der Sauerstoff abgetrennt und als Rückstrom 16 in den Chlorwasserstoff-Oxidationsreaktor zurückgeführt wird. Es wird ein Produktstrom 15 aus Chlor erhalten. Um die Aufpegelung von inerten Gasbestandteilen wie Stickstoff, Argon (gegebenenfalls aus dem Sauerstoff enthaltenden Einspeisungsstrom 3, falls kein reiner Sauerstoff eingesetzt wird) oder Kohlendioxid (gegebenenfalls aus der Verbrennung der Kohlenwasserstoffe oder Chlorkohlenwasserstoffe) zu vermeiden, ist ein Purge-Strom 16a vorgesehen.

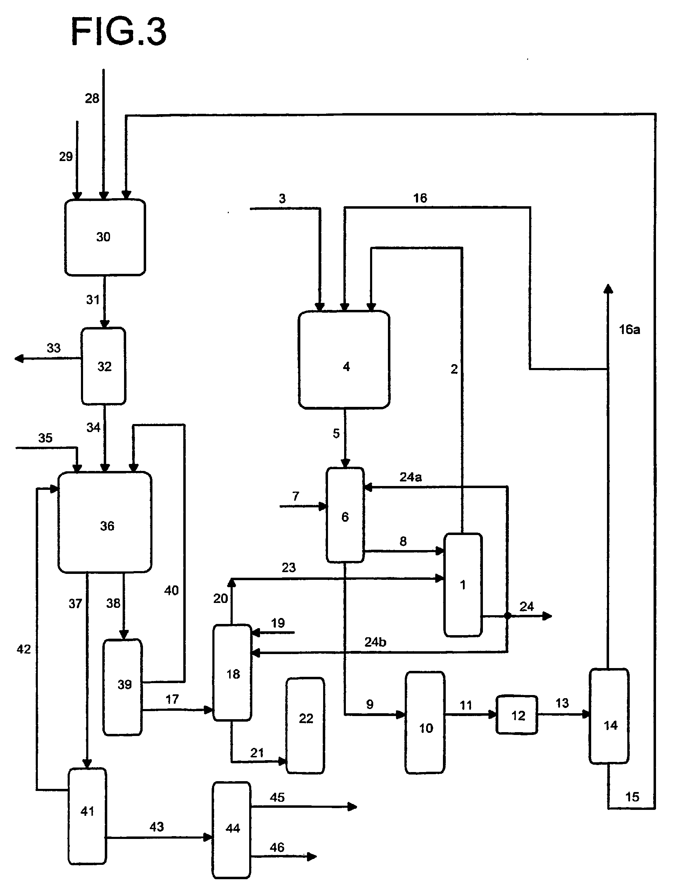

Figur 3 zeigt beispielhaft eine Variante des erfindungsgemäßen Verfahrens der Isocyanat-Herstellung.

Ein Chlor enthaltende Rückstrom 15, der aus dem Chlor-Produktstrom der Chlorwasserstoff-Oxidation erhalten wird, und gegebenenfalls durch Destillation in einer Kolonne von leichtsiedenden Nebenbestandteilen befreit worden ist, ein Chlor enthaltender Ergänzungsstrom 28 und ein Kohlenmonoxidstrom 29, bevorzugt aus einer Synthesegasanlage, werden dem Phosgensynthesereaktor 30 zugeführt und dort zu Phosgen umgesetzt, wobei Kohlenmonoxid bevorzugt im Überschuss eingesetzt wird. Der erhaltene Produktgasstrom 31, der im wesentlichen Phosgen und Kohlenmonoxid enthält und darüber hinaus noch Spuren von Chlor, Tetrachlorkohlenstoff und Inerte wie Stickstoff enthalten kann, wird der Trennstufe 32 zugeführt und dort, vorzugsweise durch Auskondensieren von Phosgen oder durch Destillation, in einen Abgasstrom 33, der im wesentlichen aus Kohlenmonoxid besteht gegebenenfalls Spuren von Chlor enthalten kann, und einen Strom 34 aus Phosgen aufgetrennt. Dieser Strom 34, ein Strom 35 aus primärem Amin, ein Phosgen-Rückstrom 40 und ein Lösemittel-Rückstrom 42 werden dem Phosgenierungsreaktor 36 zugeführt, wo die Umsetzung von Amin mit Phosgen zu Isocyanat und Chlorwasserstoff stattfindet. Der Phosgenierungsreaktor 36 kann beispielsweise als Rührkessel, Rührkessellcaskade, Reaktionskolonne oder Rohrreaktor mit vorgeschaltetem Mischorgan oder Verschaltung der vorgenannten Apparate ausgestaltet sein. Die Phosgenierung kann zweistufig als Kaltphosgenierung mit anschließender Heißphosgenierung durchgeführt werden. Es wird ein flüssiger Produktstrom 37 enthaltend Lösemittel, Isocyanat und Nebenprodukte (z. B. Harnstoff, Oligomere) erhalten, aus dem in der nachfolgenden Trennstufe 41, vorzugsweise durch Destillation, das Lösemittel abgetrennt wird. Der Lösemittelstrom 42 wird unter Ersatz von Lösemittelverlusten in den Phosgenierungsreaktor 36 zurückgeführt. Der verbleibende Isocyanatstrom 43 wird in der Reinigungsstufe 44 in Wertprodukt 45 und Schwersieder 46 aufgetrennt. Gegebenenfalls als Schwersieder anfallenden Oligomere können auch als Wertprodukt betrachtet werden. In der Phosgenierungsreaktion gebildeter Chlorwasserstoff und überschüssiges Phosgen verlassen den Phosgenierungsreaktor 36 als Gasstrom 38, der auch Lösemittelreste, leichtsiedende Nebenprodukte, Kohlenmonoxid, Kohlendioxid sowie Inertgase (beispielsweise Stickstoff, Argon) enthalten kann. Aus diesem werden in der Trennstufe 39, vorzugsweise durch Destillation, Phosgen und Lösemittelreste abgetrennt und als Rückstrom 40 in die Phosgenierungsstufe 36 zurückgeführt. Es verbleibt ein Chlorwasserstoffstrom 17, der noch geringe Mengen an Lösemittel, Phosgen oder Inerten enthalten kann. Dieser wird dem Phasenkontaktapparat 18 zugeführt und dort mit Wasser 19 in Kontakt gebracht, wobei ein Strom 21 aus verdünnter Salzsäure und ein die gasförmigen Nebenbestandteile enthaltender Abgasstrom 20 erhalten wird. Ein Teil der Salzsäure 24 kann als zusätzlicher Absorptionsmittelstrom 24b in den Phasenkontak-tapparat 18 eingespeist werden. Der Strom 21 wird in dem erfindungsgemäßen Verfahren zur Chlor-Herstellung gemäß Figur 2 eingesetzt.

## Patentansprüche

1. Verfahren zur Herstellung von Chlor aus Salzsäure mit den Schritten:
a) es wird ein Salzsäure-Einsatzstrom I bereitgestellt;
b) es wird ein Salzsäure-Rückstrom II bereitgestellt;
c) in einem Destillationsschritt wird aus dem Salzsäure-Einsatzstrom I und dem Salzsäure-Rückstrom II ein Chlorwasserstoffstrom IV abgetrennt, wobei in einem ersten Teilschritt c1) der Salzsäurestrom II in einen Chlorwasserstoffstrom IV und einen azeotrop siedenden Salzsäurestrom IIa aufgetrennt wird, und der azeotrop siedende Salzsäurestrom IIa in einem zweiten Teilschritt c2) in einen Wasserdampfstrom IX und einen Salzsäurestrom IIb höherer Konzentration als IIa aufgetrennt und der Salzsäurestrom IIb in den Teilschritt c1) zurückgeführt wird, wobei der erste Teilschritt c1) bei einem höheren Druck als der zweite Teilschritt c2) durchgeführt wird;
d) der Chlorwasserstoffstrom IV, ein Sauerstoff enthaltender Strom V und gegebenenfalls ein Sauerstoff enthaltender Rückstrom Va werden in eine Oxidationszone eingespeist und Chlorwasserstoff in Gegenwart eines Katalysators zu Chlor oxidiert, wobei ein Produktgasstrom VI erhalten wird, der Chlor, nicht umgesetzten Sauerstoff, nicht umgesetzten Chlorwasserstoff und Wasserdampf enthält;
e) aus dem Produktgasstrom VI werden in einem Absorptionsschritt Chlorwasserstoff und Wasser abgetrennt; wobei ein Gasstrom VII und der Salzsäure-Rückstrom II erhalten wird;
f) gegebenenfalls wird der Gasstrom VII getrocknet;
g) aus dem Gasstrom VII wird ein Sauerstoff enthaltender Strom abgetrennt und gegebenenfalls zumindest teilweise als Sauerstoff enthaltender Rückstrom Va in die Oxidationszone zurückgeführt, wobei ein Chlor enthaltender Produktstrom VIII verbleibt;
h) gegebenenfalls wird der Chlor enthaltende Produktstrom VIII weiter äufgereinigt.

2. Verfahren nach Anspruch 1, **dadurch gekennzeichnet, dass** der Salzsäure-Einsatzstrom I erhalten wird, indem
a1) ein Chlorwasserstoff enthaltender Einspeisungsgastrom Ia, der nicht wasserlösliche Nebenbestandteile enthalten kann, bereitgestellt wird;
a2) in einem Absorptionsschritt Chlorwasserstoff in Wasser absorbiert wird, wobei der Salzsäure-Einsatzstrom I und gegebenenfalls ein Abgasstrom III enthaltend nicht wasserlöslichen Verunreinigungen erhalten wird.

3. Verfahren nach Anspruch 1 oder 2, **dadurch gekennzeichnet, dass** der Chlorwasserstoff enthaltende Einspeisungsgasstrom Ia als Abstrom erhalten wird bei (1) der Isocyanat-Herstellung aus Phosgen und Aminen, (2) der Säurechlorid-Herstellung, (3) der Polycarbonat-Herstellung, (4) der Herstellung von Vinylchlorid aus Ethylendichlorid und/oder der Chlorierung von Aromaten (5).

4. Verfahren nach einem der Ansprüche 1-3, **dadurch gekennzeichnet, dass** der Druck im ersten Teilschritt 1 bis 20 bar beträgt.

5. Verfahren zur Herstellung von organischen Isocyanaten mit den Schritten
i) ein Kohlenmonoxid enthaltender Einspeisungsgassstrom X, ein Chlor enthaltender Rückstrom VIII und gegebenenfalls ein Chlor enthaltender Ergänzungsstrom VIIIa werden bereitgestellt;
ii) in einem Phosgen-Syntheseschritt werden die Ströme X, VIII und gegebenenfalls VIIIa zu einem Phosgen enthaltenden Gasstrom XI umgesetzt;
iii) in einem Isocyant-Syntheseschritt wird der Phosgen enthaltende Gastrom XI mit einem oder mehreren primären Aminen zu dem oder den entsprechenden Isocyanaten und Chlorwasserstoff umgesetzt, wobei ein Chlorwasserstoff und nicht umgesetztes Phosgen enthaltender Gasstrom XII und ein Isocyanat enthaltender Strom Produktstrom XIII gewonnen werden;
iv) aus dem Chlorwasserstoff und nicht umgesetztes Phosgen enthaltenden Gasstrom XII wird ein einem Auftrennschritt ein Chlorwasserstoff und gegebenenfalls nicht wasserlösliche Verunreinigungen enthaltender Gasstrom Ia und ein Phosgen enthaltender Strom XIV gewonnen, wobei der Phosgen enthaltende Strom XIV gegebenenfalls in den Isocyanat-Syntheseschritt iii) zurückgeführt wird;
v) in einem Absorptionsschritt wird Chlorwasserstoff aus dem Gasstrom Ia in Wasser absorbiert, wobei ein Salzsäure-Einsatzstrom I aus verdünnter Salzsäure und gegebenenfalls ein Abgasstrom III enthaltend die nicht wasserlöslichen Verunreinigungen erhalten wird;
vi) aus dem Salzsäure-Einsatzstrom I und einem Salzsäure-Rückstrom II wird in einem Destillationsschritt ein Chlorwasserstoffstrom IV abgetrennt, wobei in einem ersten Teilschritt vi-1) der Salzsäure-Einsatzstrom I und der Salzsäure-Rückstromstrom II in einen Chlorwasserstoffstrom IV und einen azeotrop siedenden Salzsäurestrom IIa aufgetrennt wird, und der azeotrop siedende Salzsäurestrom IIa in einem zweiten Teilschritt vi-2) in einen Wasserdampfstrom IX und einen Salzsäurestrom IIb höherer Konzentration als IIa aufgetrennt und der Salzsäurestrom IIb in den Teilschritt vi-1) zurückgeführt wird, wobei der erste Teilschritt vi-1) bei einem höheren Druck als der zweite Teilschritt vi-2) durchgeführt wird;
vii)der Chlorwasserstoffstrom IV, ein Sauerstoff enthaltender Strom V und gegebenenfalls ein Sauerstoff enthaltender Rückstrom Va werden in eine Oxidationszone eingespeist und Chlorwasserstoff in Gegenwart eines Katalysators zu Chlor oxidiert, wobei ein Produktgasstrom VI erhalten wird, der Chlor, nicht umgesetzten Sauerstoff, nicht umgesetzten Chlorwasserstoff und Wasserdampf enthält;
viii)aus dem Produktgasstrom VI werden in einem Absorptionsschritt Chlorwasserstoff und Wasser abgetrennt, wobei ein Gasstrom VII und ein Salzsäure-Rückstrom II aus verdünnter Salzsäure erhalten wird;
ix) der Gasstrom VII wird getrocknet;
x) aus dem Gasstrom VII wird ein Sauerstoff enthaltender Strom abgetrennt und gegebenenfalls zumindest teilweise als Sauerstoff enthaltender Rückstrom Va in die Oxidationszone zurückgeführt, wobei ein Chlor enthaltender Produktstrom VIII verbleibt;
xi) der Chlor enthaltende Produktstrom VIII wird, gegebenenfalls nach Aufreinigung, als Chlor enthaltender Rückstrom VIII in Schritt i) eingesetzt.

## Claims

1. A process for preparing chlorine from hydrochloric acid, which comprises the steps:
a) providing a hydrochloric acid feed stream I;
b) providing a hydrochloric acid recycle stream II;
c) separating off a hydrogen chloride stream IV from the hydrochloric acid feed stream I and the hydrochloric acid recycle stream II in a distillation step, where the hydrochloric acid stream II is fractionated in a first substep c1) to give a hydrogen chloride stream IV and an azeotropic hydrochloric acid stream IIa and the azeotropic hydrochloric acid stream IIa is fractionated in a second substep c2) to give a water vapor stream IX and a hydrochloric acid stream IIb which has a concentration higher than that of IIa and the hydrochloric acid stream IIb is recirculated to the substep c1), with the first substep c1) being carried out at a higher pressure than the second substep c2);
d) feeding the hydrogen chloride stream IV, an oxygen-comprising stream V and, if desired, an oxygen-comprising recycle stream Va into an oxidation zone and oxidizing hydrogen chloride to chlorine in the presence of a catalyst to give a product gas stream VI comprising chlorine, unreacted oxygen, unreacted hydrogen chloride and water vapor;
e) separating off hydrogen chloride and water from the product gas stream VI in an absorption step to give a gas stream VII and the hydrochloric acid recycle stream II;
f) if desired, drying the gas stream VII;
g) separating off an oxygen-comprising stream from the gas stream VII and, if desired, recirculating at least part of this as oxygen-comprising recycle stream Va to the oxidation zone, leaving a chlorine-comprising product stream VIII;
h) if desired, further purifying the chlorine-comprising product stream VIII.

2. The process according to claim 1, wherein the hydrochloric acid feed stream I is obtained by
a1) preparing a feed gas stream Ia which comprises hydrogen chloride and may comprise secondary constituents which are not soluble in water;
a2) absorbing hydrogen chloride in water in an absorption step to give the hydrochloric acid feed stream I and possibly an offgas stream III comprising impurities which are not soluble in water.

3. The process according to claim 1 or 2, wherein the feed gas stream Ia comprising hydrogen chloride is obtained as offgas stream in (1) isocyanate production from phosgene and amines, (2) acid chloride production, (3) polycarbonate production, (4) preparation of vinyl chloride from ethylene dichloride and/or chlorination of aromatics (5).

4. The process according to any of claims 1-3, wherein the pressure in the first substep is from 1 to 20 bar.

5. A process for preparing organic isocyanates, which comprises the steps
i) providing a feed gas stream X comprising carbon monoxide, a chlorine-comprising recycle stream VIII and, if desired, a chlorine-comprising supplementary stream VIIIa;
ii) reacting the streams X, VIII and, if used, VIIIa in a phosgene synthesis step to give a phosgene-comprising gas stream XI;
iii) reacting the phosgene-comprising gas stream XI with one or more primary amines in an isocyanate synthesis step to form the corresponding isocyanate(s) and hydrogen chloride and give a gas stream XII comprising hydrogen chloride and unreacted phosgene and an isocyanate-comprising product stream XIII;
iv) fractionating the gas stream XII comprising hydrogen chloride and unreacted phosgene in a fractionation step to give a gas stream Ia comprising hydrogen chloride and possibly impurities which are not soluble in water and a phosgene-comprising stream XIV, and, if desired, recirculating the phosgene-comprising stream XIV to the isocyanate synthesis step iii);
v) absorbing hydrogen chloride from the gas stream Ia in water in an absorption step to give a hydrochloric acid feed stream I comprising dilute hydrochloric acid and possibly an offgas stream III comprising the impurities which are not soluble in water;
vi) separating off a hydrogen chloride stream IV from the hydrochloric acid feed stream I and a hydrochloric acid recycle stream II in a distillation step, where the hydrochloric acid feed stream I and the hydrochloric acid recycle stream II are fractionated in a first substep vi-1) to give a hydrogen chloride stream IV and an azeotropic hydrochloric acid stream IIa and the azeotropic hydrochloric acid stream IIa is fractionated in a second substep vi-2) to give a water vapor stream IX and a hydrochloric acid stream IIb which has a concentration higher than that of IIa and the hydrochloric acid stream IIb is recirculated to the substep vi-1), with the first substep vi-1) being carried out at a higher pressure than the second substep vi-2);
vii) feeding the hydrogen chloride stream IV, an oxygen-comprising stream V and, if desired, an oxygen-comprising recycle stream Va into an oxidation zone and oxidizing hydrogen chloride in the presence of a catalyst to form chlorine and give a product gas stream VI comprising chlorine, unreacted oxygen, unreacted hydrogen chloride and water vapor;
viii) separating off hydrogen chloride and water from the product gas stream VI in an absorption step to give a gas stream VII and a hydrochloric acid recycle stream II comprising dilute hydrochloric acid;
ix) drying the gas stream VII;
x) separating off an oxygen-comprising stream from the gas stream VII and, if desired, recirculating at least part of this as oxygen-comprising recycle stream Va to the oxidation zone to leave a chlorine-comprising product stream VIII;
xi) using the chlorine-comprising product stream VIII, if desired after purification, as chlorine-comprising recycle stream VIII in step i).

## Revendications

1. Procédé de fabrication de chlore à partir d'acide chlorhydrique selon les étampes :
a) un courant d'alimentation d'acide chlorhydrique I est préparé ;
b) un courant de recyclage d'acide chlorhydrique II est préparé ;
c) lors d'une étape de distillation, un courant de chlorure d'hydrogène IV est séparé à partir du courant d'alimentation d'acide chlorhydrique I et du courant de recyclage d'acide chlorhydrique II, le courant d'acide chlorhydrique II étant séparé lors d'une première étape partielle c1) en un courant de chlorure d'hydrogène IV et un courant d'acide chlorhydrique à ébullition azéotropique IIa, et le courant d'acide chlorhydrique à ébullition azéotropique IIa étant séparé lors d'une seconde étape partielle c2) en un courant de vapeur d'eau IX et un courant d'acide chlorhydrique IIb de concentration supérieure à IIa, et le courant d'acide chlorhydrique IIb étant recyclé dans l'étape partielle c1), la première étape partielle c1) étant réalisée à une pression supérieure à la seconde étape partielle c2) ;
d) le courant de chlorure d'hydrogène IV, un courant V contenant de l'oxygène et éventuellement un courant de recyclage Va contenant de l'oxygène sont introduits dans une zone d'oxydation et le chlorure d'hydrogène est oxydé en chlore en présence d'un catalyseur, un courant gazeux de produits VI étant obtenu, qui contient du chlore, de l'oxygène non réagi, du chlorure d'hydrogène non réagi et de la vapeur d'eau ;
e) le chlorure d'hydrogène et l'eau sont séparés du courant gazeux de produits VI lors d'une étape d'absorption ; un courant gazeux VII et le courant de recyclage d'acide chlorhydrique II étant obtenus ;
f) le courant gazeux VII est éventuellement séché ;
g) un courant contenant de l'oxygène est séparé du courant gazeux VII et éventuellement recyclé au moins en partie en tant que courant de recyclage Va contenant de l'oxygène dans la zone d'oxydation, un courant de produits contenant du chlore VIII demeurant ;
h) le courant de produits contenant du chlore VIII est éventuellement davantage purifié.

2. Procédé selon la revendication 1, **caractérisé en ce que** le courant d'alimentation d'acide chlorhydrique I est obtenu par les étampes :
a1) un courant gazeux d'alimentation Ia contenant du chlorure d'hydrogène, qui peut contenir des constituants secondaires non solubles dans l'eau, est préparé ;
a2) le chlorure d'hydrogène est absorbé dans de l'eau lors d'une étape d'absorption, le courant d'alimentation d'acide chlorhydrique I et éventuellement un courant de gaz d'échappement III contenant des impuretés non solubles dans l'eau étant obtenus.

3. Procédé selon la revendication 1 ou 2, **caractérisé en ce que** le courant gazeux d'alimentation Ia contenant du chlorure d'hydrogène est obtenu en tant que courant de déchargement lors (1) de la fabrication d'isocyanate à partir de phosgène et d'amines, (2) de la fabrication de chlorure d'acide, (3) de la fabrication de polycarbonate, (4) de la fabrication de chlorure de vinyle à partir de dichlorure d'éthylène et/ou de la chloration de composés aromatiques (5).

4. Procédé selon l'une quelconque des revendications 1 à 3, **caractérisé en ce que** la pression lors de la première étape partielle est de 1 à 20 bar.

5. Procédé de fabrication d'isocyanates organiques selon les étampes :
i) un courant gazeux d'alimentation X contenant du monoxyde de carbone, un courant de recyclage VIII contenant du chlore et éventuellement un courant complémentaire VIIIa contenant du chlore sont préparés ;
ii) les courants X, VIII et éventuellement VIIIa sont transformés en un courant gazeux XI contenant du phosgène lors d'une étape de synthèse de phosgène ;
iii) lors d'une étape de synthèse d'isocyanate, le courant gazeux XI contenant du phosgène est mis en réaction avec une ou plusieurs amines primaires pour former le ou les isocyanates correspondants et du chlorure d'hydrogène, un courant gazeux XII contenant du chlorure d'hydrogène et du phosgène non réagi et un courant de produits XIII contenant de l'isocyanate étant obtenus ;
iv) à partir du courant gazeux XII contenant du chlorure d'hydrogène et du phosgène non réagi, un courant gazeux Ia contenant du chlorure d'hydrogène et éventuellement des impuretés non solubles dans l'eau et un courant XIV contenant du phosgène sont obtenus lors d'une étape de séparation, le courant XIV contenant du phosgène étant éventuellement recyclé dans l'étape de synthèse d'isocyanate iii) ;
v) le chlorure d'hydrogène du courant gazeux Ia est absorbé dans de l'eau lors d'une étape d'absorption, un courant d'alimentation d'acide chlorhydrique I constitué d'acide chlorhydrique dilué et éventuellement un courant de gaz d'échappement III contenant les impuretés non solubles dans l'eau étant obtenus ;
vi) un courant de chlorure d'hydrogène IV est séparé à partir du courant d'alimentation d'acide chlorhydrique I et d'un courant de recyclage d'acide chlorhydrique II lors d'une étape de distillation, le courant d'alimentation d'acide chlorhydrique I et le courant de recyclage d'acide chlorhydrique II étant séparés lors d'une première étape partielle vi-1) en un courant de chlorure d'hydrogène IV et un courant d'acide chlorhydrique à ébullition azéotropique IIa, et le courant d'acide chlorhydrique à ébullition azéotropique IIa étant séparé lors d'une seconde étape partielle vi-2) en un courant de vapeur d'eau IX et un courant d'acide chlorhydrique IIb de concentration supérieure à IIa, et le courant d'acide chlorhydrique IIb étant recyclé dans l'étape partielle vi-1), la première étape partielle vi-1) étant réalisée à une pression supérieure à la seconde étape partielle vi-2) ;
vii) le courant de chlorure d'hydrogène IV, un courant V contenant de l'oxygène et éventuellement un courant de recyclage Va contenant de l'oxygène sont introduits dans une zone d'oxydation et le chlorure d'hydrogène est oxydé en chlore en présence d'un catalyseur, un courant gazeux de produits VI étant obtenu, qui contient du chlore, de l'oxygène non réagi, du chlorure d'hydrogène non réagi et de la vapeur d'eau ;
viii) le chlorure d'hydrogène et l'eau sont séparés du courant gazeux de produits VI lors d'une étape d'absorption ; un courant gazeux VII et le courant de recyclage d'acide chlorhydrique II constitué d'acide chlorhydrique dilué étant obtenus ;
ix) le courant gazeux VII est séché ;
x) un courant contenant de l'oxygène est séparé du courant gazeux VII et éventuellement recyclé au moins en partie en tant que courant de recyclage Va contenant de l'oxygène dans la zone d'oxydation, un courant de produits VIII contenant du chlore demeurant ;
xi) le courant de produits VIII contenant du chlore est utilisé en tant que courant de recyclage VIII contenant du chlore dans l'étape i), éventuellement après une purification.
